# EUROPÄISCHE PATENTANMELDUNG

(11) **EP 4 656 209 A1**
(43) Veröffentlichungstag der Anmeldung: **03.12.2025**
(21) Anmeldenummer: 24178364.6
(22) Anmeldetag: 28.05.2024
(51) Int. Cl.: A61L 2/20

(54) **VERFAHREN ZUM ÜBERFÜHREN VON OBJEKTEN AUS EINER SCHLEUSE IN DIE ARBEITSKAMMER EINES CONTAINMENTS UNTER ASEPTISCHEN BEDINGUNGEN**

(71) Anmelder: SKAN AG, 4123 Allschwil (CH)
(72) Erfinder: Hillbrand, Sebastian, 4222 Zwingen (CH); Kemmerling, Simon, 4106 Therwil (CH); Lehmann, Frank Martin, 4102 Binningen (CH); Mittelviefhaus, Maximilian, 4052 Basel (CH); Sommerhalder, Matthias, 4112 Bättwil (CH)
(74) Vertreter: Ullrich, Gerhard

(57) **Zusammenfassung**

Die vorliegende Erfindung betrifft ein Verfahren zum Überführen von Objekten (**4**) unter aseptischen Bedingungen aus einer Schleuse (**3**) in die von einem Gehäuse (**20**) umgebene Prozesskammer (**21**) eines Containments (**2**), das mit der Schleuse (**3**) eine druckdicht miteinander verbundene Transfereinheit (**1**) bildet. Ein Gehäuse (**30**) der Schleuse (**3**) umgibt eine Schleusenkammer (**31**) mit dem Schleusenvolumen (**V_{S}**). Die Schleusenkammer (**31**) besitzt ein druckdicht verschliessbares Eingangstor (**37**), das im offenen Zustand einen Zugang (**33**) vom äusseren Aufstellraum (**A**) zum Einbringen von Objekten (**4**) in die Schleusenkammer (**31**) erlaubt. Ferner weist die Schleuse (**3**) einen Ausgang (**38**) auf, der zum Überführen der Objekte (**4**) aus der Schleusenkammer (**31**) in die Prozesskammer (**21**) bestimmt ist. Ausserdem hat die Schleuse (**3**) einen Einlass (**34**) zum Einbringen von Dekontaminationsmittel.

## Beschreibung

### Anwendungsgebiet der Erfindung

Die vorliegende Erfindung betrifft ein Verfahren zum Überführen von Objekten unter aseptischen Bedingungen aus einer Schleuse in die von einem Gehäuse umgebene Prozesskammer eines Containments, das mit der Schleuse eine druckdicht miteinander verbundene Transfereinheit bildet.

### Stand der Technik

Der zum oben definierten Anwendungsgebiet vorbekannte Stand der Technik ist im Patentschrifttum gemäss EP 3 444 193 A1, US 10,744,659 B2 und der CN 219 638 704 U offenbart.

### Aufgabe der Erfindung

Das Ziel der vorliegenden Erfindung besteht darin, ein effizienteres Verfahren zum Überführen von Objekten unter aseptischen Bedingungen aus einer Schleuse in die von einem Gehäuse umgebene Prozesskammer eines Containments, das mit der Schleuse eine druckdicht miteinander verbundene Transfereinheit bildet, vorzuschlagen. Hierbei soll das Überführen der Objekte in möglichst kurzer Zeit erfolgen und dabei die Transfereinheit sich insgesamt gerätetechnisch mit geringem Aufwand flexibel gestalten lassen, um variierenden Anforderungen gerecht zu werden.

### Übersicht über die Erfindung

Das erfindungsgemässe Verfahren zum Überführen von Objekten unter aseptischen Bedingungen aus einer Schleuse in die von einem Gehäuse umgebene Prozesskammer eines Containments, das mit der Schleuse eine druckdicht miteinander verbundene Transfereinheit bildet, beruht darauf, dass:
- ein Gehäuse der Schleuse eine Schleusenkammer mit dem Schleusenvolumen umgibt;
- die Schleusenkammer besitzt:
   - ein druckdicht verschliessbares Eingangstor, welches im offenen Zustand einen Zugang vom äusseren Aufstellraum zum Einbringen von Objekten in die Schleusenkammer erlaubt;
   - einen Ausgang, der zum Überführen der Objekte aus der Schleusenkammer in die Prozesskammer bestimmt ist; und
   - einen Einlass zum Einbringen von Dekontaminationsmittel;
- das Gehäuse einen Zugang in die Prozesskammer aufweist;
- der Ausgang aus der Schleusenkammer in den Zugang in die Prozesskammer mündet;
- zwischen dem Ausgang aus der Schleusenkammer und dem Zugang in die Prozesskammer eine verschliessbare Transfertür vorgesehen ist; und
- ein einzelnes Objekt umfasst:
   - ein Behältnis mit einem aseptischen Innenraum und einer Öffnung, die mit einem Verschluss verschlossen ist; und
   - im Innenraum gelagertes Material, welches nach Aufmachen des Verschlusses in der Prozesskammer zu behandeln ist.

Kennzeichen sind folgende Verfahrensschritte:
- Bestimmung von Dimension und Form der Schleusenkammer adäquat zum äusseren Objektvolumen eines einzelnen oder mehrerer Objekte mit dem Ziel der Minimierung des durch das eine oder mehrerer im Schleusenvolumen liegenden Objekte verbleibenden freien Schleusenvolumens;
- Einbringen eines einzelnen oder mehrerer Objekte in die ausgewählte Schleusenkammer durch das offene Eingangstor bei geschlossener Transfertür;
- Einleiten von Dekontaminationsmittel über den Einlass in die Schleusenkammer bei geschlossenem Eingangstor;
- optionales Spülen der Schleusenkammer und Ableiten von Spülmittel aus der Schleusenkammer über einen Auslass nach erfolgreicher Dekontamination; und
- Überführen des einzelnen oder mehrerer äusserlich dekontaminierter Objekte durch die geöffnete Transfertür aus der Schleusenkammer in die Prozesskammer.

Nachfolgend werden spezielle Ausführungsformen der Erfindung definiert:
Aufgrund des geringen verbleibenden freien Schleusenvolumens kann die Dekontaminationsphase zeitlich kurzgehalten werden, somit wird das Diffundieren von Dekontaminationsmittel in den Innenraum des Objekts minimiert und die Verarbeitung des Objekts im Containment erfolgt rasch.

Die Verbindung zwischen der Schleuse und dem Containment erfolgt mittels beiderseits vorhandener Flanschanordnungen, welche vorzugsweise druckdicht ausgestaltet sind.

Das Dekontaminationsmittel in der Schleusenkammer wird zerstäubt, z.B. mittels einer Zweistoffdüse oder durch Ultraschall, und ist vorzugsweise flüssiges H₂O₂.

Das optionale Spülen der Schleusenkammer erfolgt durch alternierenden Fluss in wechselnder Strömungsrichtung aus den Gasvolumen zwischen der Prozesskammer und der Schleusenkammer.

Die Transfertür ist am Ausgang aus der Schleusenkammer oder am Zugang in das Containment vorgesehen.

Die an der Schleuse und am Containment vorhandenen Flanschanordnungen sind als Schnittstelle beschaffen, wodurch das Andocken verschieden dimensionierter Schleusen unterschiedlicher Formen an das Containment ermöglicht wird.

Das Überführen des einzelnen oder mehrerer äusserlich dekontaminierter Objekte aus der Schleusenkammer in die Prozesskammer erfolgt nach teilweiser oder vollständiger Spülung der Schleusenkammer.

Vor dem Beladen der Schleuse werden bei geschlossenem Eingangstor und offener Transfertür die Schleusenkammer und die Prozesskammer sowie die Dichtflächen an der Transfertür dekontaminiert.

Die Schleuse besitzt einen Anschluss, um daran einen Medienblock anzuschliessen, welcher mit einer Prozesstechnik verbunden ist, die Bestandteil des Containments oder der Schleuse sein kann oder unabhängig davon positioniert ist.

Der Medienblock weist Leitungen für Druckluft und/oder Dekontaminationsmittel, und/oder Sensoren für Temperatur, Feuchte, Druck, H₂O₂-Konzentration und Prozess- und Abluft auf.

Das optionale Spülen erfolgt mittels Druckluft und/oder Prozessluft, welche über die Umgebungstemperatur im Aufstellraum hinaus aufgewärmt sein kann.

Die Verbindung zwischen dem Anschluss an der Schleuse und dem Medienblock ist mechanisch lösbar oder unlösbar oder pneumatisch gekoppelt beschaffen.

Nach Einbringen eines einzelnen oder mehrerer Objekte in die Schleusenkammer beträgt gemäss deren gewählter Dimensionierung das vom Schleusenvolumen verbleibende freie Schleusenvolumen zwischen 10% und 80%, vorzugsweise zwischen 20% und 60%, insbesondere zwischen 30% und 50%.

Die Anpassung der Dimensionierung des Schleusenvolumen an das einzubringende einzelne oder mehrerer Objekte, mit der Zielstellung der Minimierung des verbleibenden freien Schleusenvolumens wird auf zwischen 10% und 80%, vorzugsweise auf zwischen 20% und 60%, insbesondere auf zwischen 30% und 50% durch einengendes Verschieben von Wandungen an der Schleusenkammer und/oder durch Einbringen eines Volumen verdrängenden Füllkörpers realisiert.

Das Objekt hat die Gestalt eines Gebindes mit seinem äusseren Objektvolumen und umfasst:
- ein Behältnis mit einem aseptischen Innenraum und einer Öffnung, die mit einem Verschluss, vorzugsweise in Gestalt einer Abdeckung, verschlossen ist; und
- im Innenraum gelagertes Material in Form von Phiolen oder medizinischen Spritzen, welche nach Aufmachen des Verschlusses in der Prozesskammer zu behandeln sind, z.B. mit pharmazeutischen Substanzen zu befüllen sind.

Alternativ hat das Objekt die Gestalt einer Flasche, z.B. einer Zellkulturflasche, oder eines Beutels - z.B. mit darin enthaltenen Agarplatten - oder einer Ampulle hat, welche ein äusseres Objektvolumen bilden und umfasst:
- einen aseptischen Innenraum und einer vorhandenen Öffnung, die mit einem Verschluss verschlossen ist oder erst zu schaffen ist; und
- im Innenraum gelagertes Material, welches nach Aufmachen des Verschlusses in der Prozesskammer zu behandeln ist; oder
- den zu befüllenden Innenraum, z.B. mit pharmazeutischen Substanzen.

Das Spülen der Schleusenkammer erfolgt bei offener Transfertür durch mindestens ein einmaliges und zumindest partielles Einfahren des Objekts aus der Prozesskammer des Containments in die Schleusenkammer.

Zur Beschleunigung des Spülens der Schleusenkammer ist eine an diese aktivierbare Vakuumpumpe angeschlossen.

Bei beschleunigtem Spülen der Schleusenkammer:
- wird zuerst ein an die Schleusenkammer angeschlossener Auslass geöffnet, der zu einem Gasbehälter führt; und anschliessend
- wird die an den Gasbehälter angeschlossene Vakuumpumpe aktiviert; oder
- wird zuerst die an den Gasbehälter angeschlossene Vakuumpumpe aktiviert; und anschliessend
- wird ein an die Schleusenkammer angeschlossener Auslass geöffnet, der zu einem Gasbehälter führt.

Alternativ bei beschleunigtem Spülen der Schleusenkammer:
- wird zuerst der an die Schleusenkammer angeschlossene Auslass geöffnet, der zu einem Gasbehälter führt; und anschliessend
- wird die an den Gasbehälter angeschlossene Vakuumpumpe aktiviert; und
- dann wird zum Druckausgleich zwischen der Prozesskammer und der Schleusenkammer die Dichtung an der Transfertür gelöst; oder
- wird zuerst die an den Gasbehälter angeschlossene Vakuumpumpe aktiviert; und anschliessend
- wird der an die Schleusenkammer angeschlossene Auslass geöffnet, der zu einem Gasbehälter führt; und
- dann wird zum Druckausgleich zwischen der Prozesskammer und der Schleusenkammer die Dichtung an der Transfertür gelöst.

Das Lösen der Dichtung an der Transfertür zwecks Druckausgleichs zwischen Prozesskammer und Schleusenkammer erfolgt durch Deaktivierung einer pneumatischen Dichtung.

### Kurzbeschreibung der beigefügten Zeichnungen

Es zeigen:

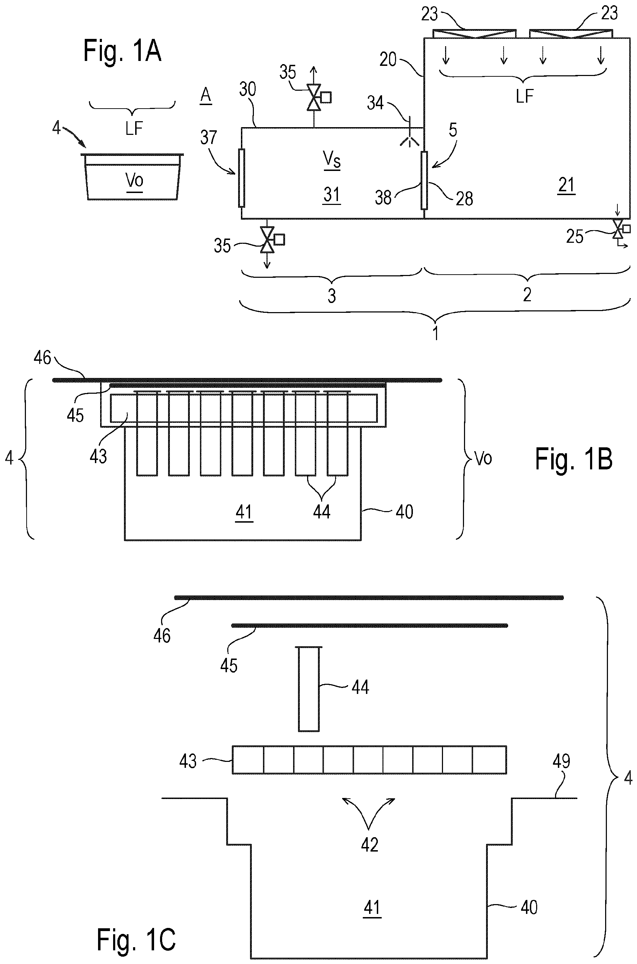

- Figur 1A -: eine Transfereinheit mit einem Containment vorgesetzter Schleuse und dieser angenähertem, zu verarbeitendem Objekt, in Prinzipdarstellung;
- Figur 1B -: das Objekt aus Figur 1A in Gestalt eines Gebindes;
- Figur 1C -: das Objekt gemäss Figur 1B, in Explosivdarstellung;
- Figur 2A -: eine Transfereinheit, bestehend aus dem Containment und einer daran andockbaren Schleuse *erster Gestalt;*
- Figur 2B -: eine Transfereinheit, bestehend aus dem Containment und einer daran andockbaren Schleuse *zweiter Gestalt;*
- Figur 2C -: eine Transfereinheit, bestehend aus dem Containment und einer daran andockbaren Schleuse *dritter Gestalt;*
- Figur 3A -: die Transfereinheit gemäss Figur 2A mit bemasster Schleuse;
- Figur 3B -: eine Transfereinheit, bestehend aus dem Containment und einer daran andockbaren Schleuse *vierter Gestalt;*
- Figur 3C -: eine Transfereinheit, bestehend aus dem Containment und einer daran andockbaren Schleuse *fünfter Gestalt;*

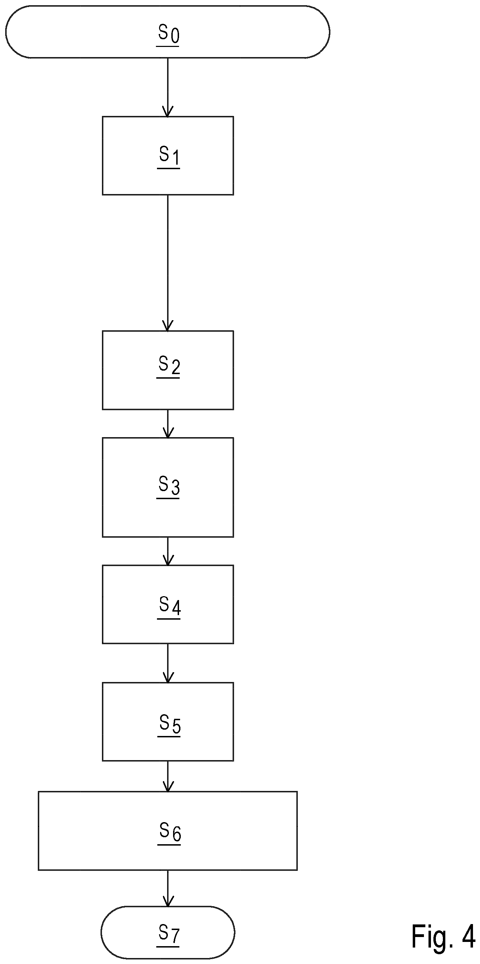

- Figur 4 -: der mittels einer Transfereinheit durchgeführte Prozessablauf zur Verarbeitung eines Objekts, dargestellt als Fliessschema;

Figuren 5A bis 5G: der mittels einer Transfereinheit durchgeführte Prozessablauf zur Verarbeitung eines Objekts, in Schrittfolge mit Prinzipdarstellungen;

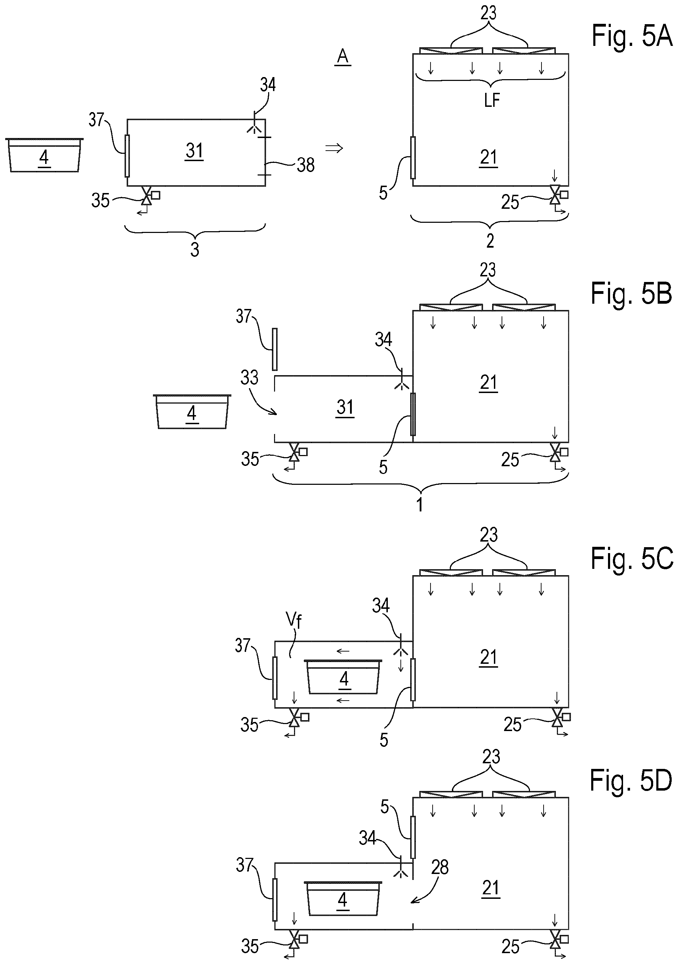

- Figur 5A -: Start: mit Schritt 1
- Figur 5B -: Schritt 2
- Figur 5C -: Schritt 3
- Figur 5D -: Schritt 4

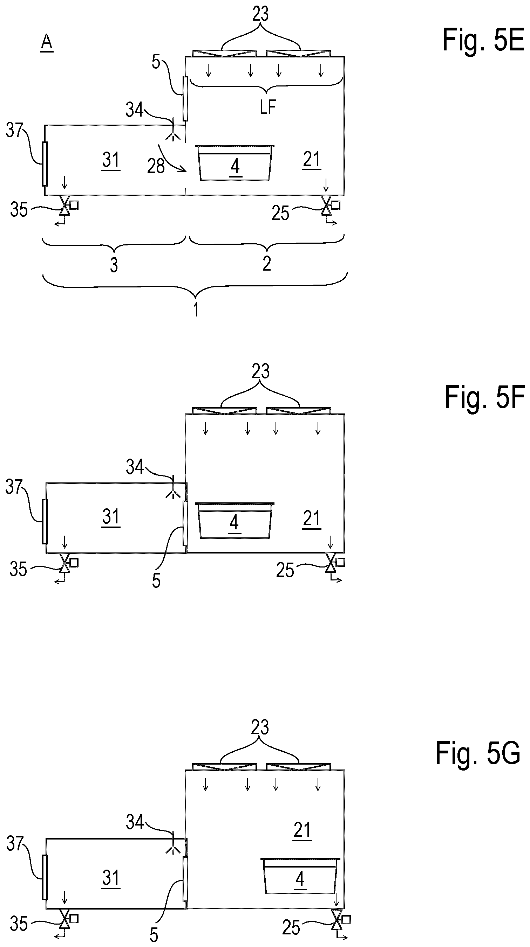

- Figur 5E -: Schritt 5
- Figur 5F -: Schritt 6
- Figur 5G -: Ende: mit Schritt 7

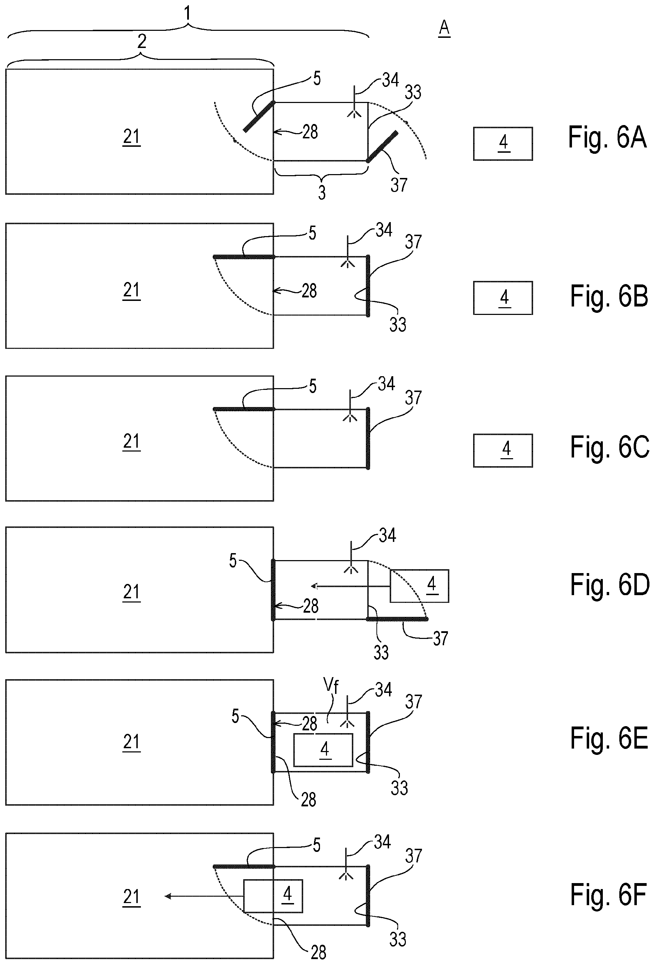

Figuren 6A bis 6F: der mittels einer Transfereinheit durchgeführte Prozessablauf zur Verarbeitung eines Objekts, mit Bewegungen des Objekts und Stellung von Eingangstor an der Schleuse und Transfertür am Übergang von der Schleuse zum Containment, in Schrittfolge mit Prinzipdarstellungen;
- Figur 6A -: Start: mit Schritt 1
- Figur 6B -: Schritt 2
- Figur 6C -: Schritt 3
- Figur 6D -: Schritt 4
- Figur 6E -: Schritt 5
- Figur 6F -: Ende: mit Schritt 6

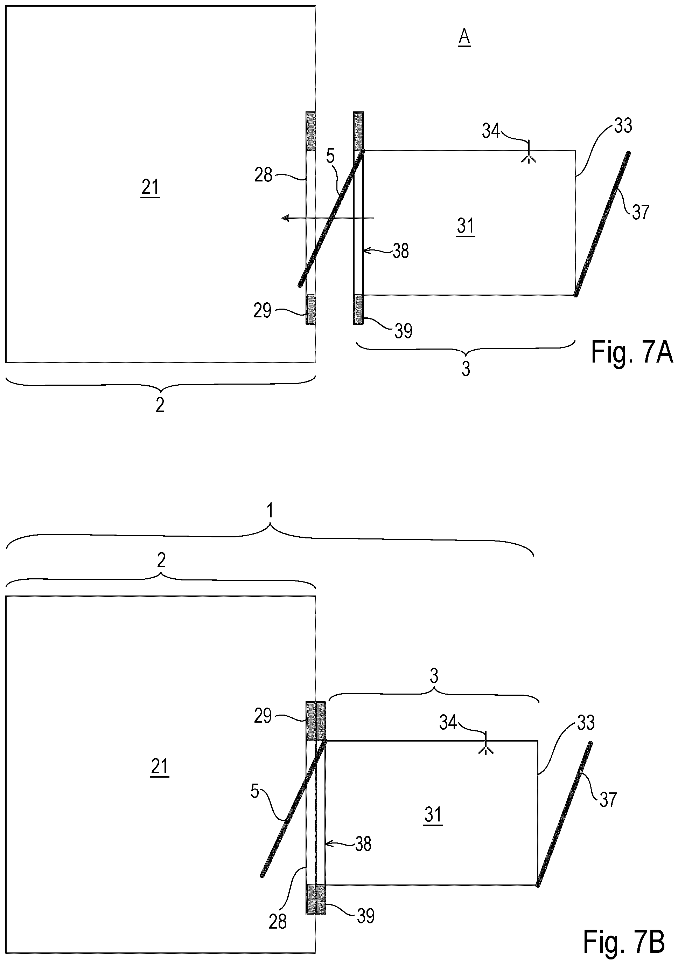

Figuren 7A und 7B: den Zusammenbau von Schleuse und Containment, in Prinzipdarstellungen;
- Figur 7A -: die Annäherung der Schleuse an das Containment;
- Figur 7B -: die Schleuse an das Containment lösbar angedockt;

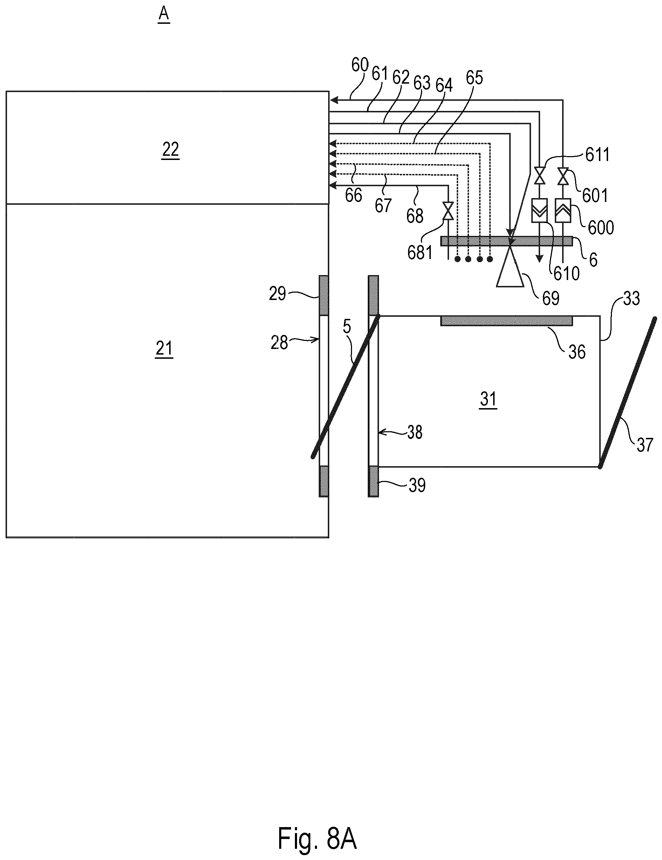

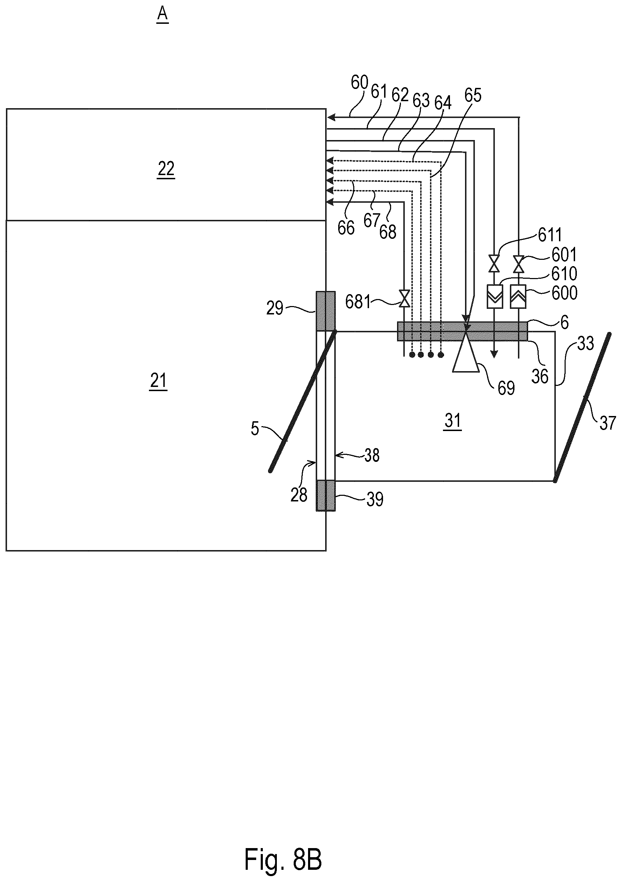

Figuren 8A und 8B: den Zusammenbau von Schleuse und Containment, ausgestattet mit einem Medienblock, in Prinzipdarstellungen;
- Figur 8A -: die Annäherung der Schleuse an das Containment, Medienblock und Anschluss noch getrennt;
- Figur 8B -: die Schleuse an das Containment lösbar angedockt, Medienblock und Anschluss miteinander verbunden;

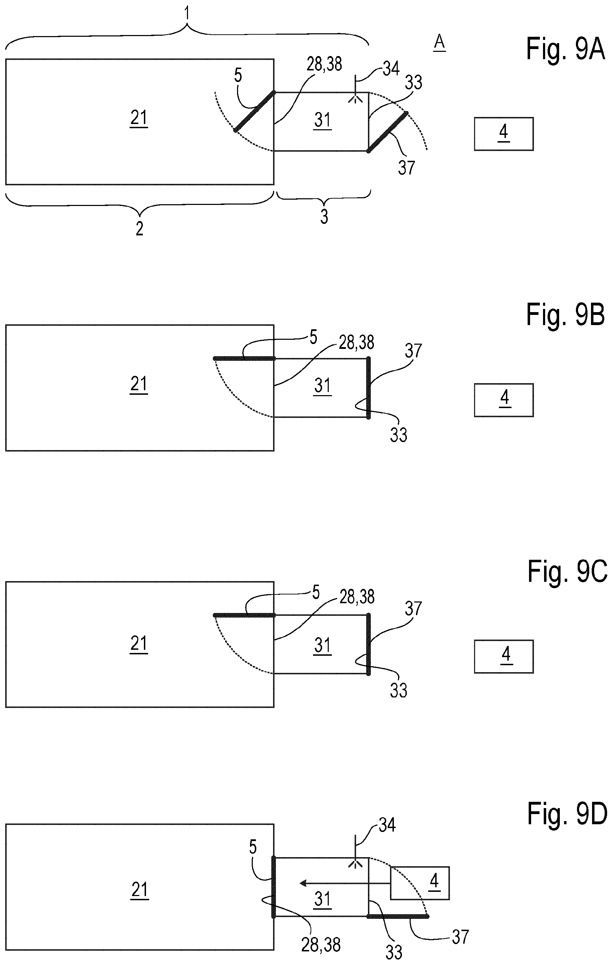

Figuren 9A bis 9G: der mittels einer Transfereinheit durchgeführte Prozessablauf zur Verarbeitung eines Objekts, mit Bewegungen des Objekts, Stellung von Eingangstor an der Schleuse und Transfertür am Übergang von der Schleuse zum Containment, mit Dekontaminations- und Spülvorgang, in Schrittfolge mit Prinzipdarstellungen;
- Figur 9A -: Start: mit Schritt 1
- Figur 9B -: Schritt 2
- Figur 9C -: Schritt 3
- Figur 9D -: Schritt 4

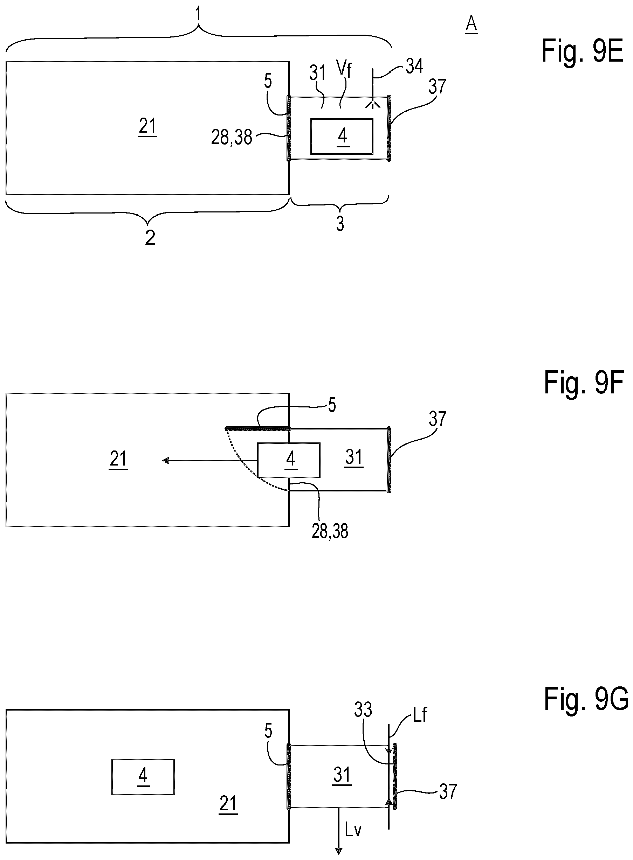

- Figur 9E -: Schritt 5
- Figur 9F -: Schritt 6; und
- Figur 9G -: Ende: mit Schritt 7

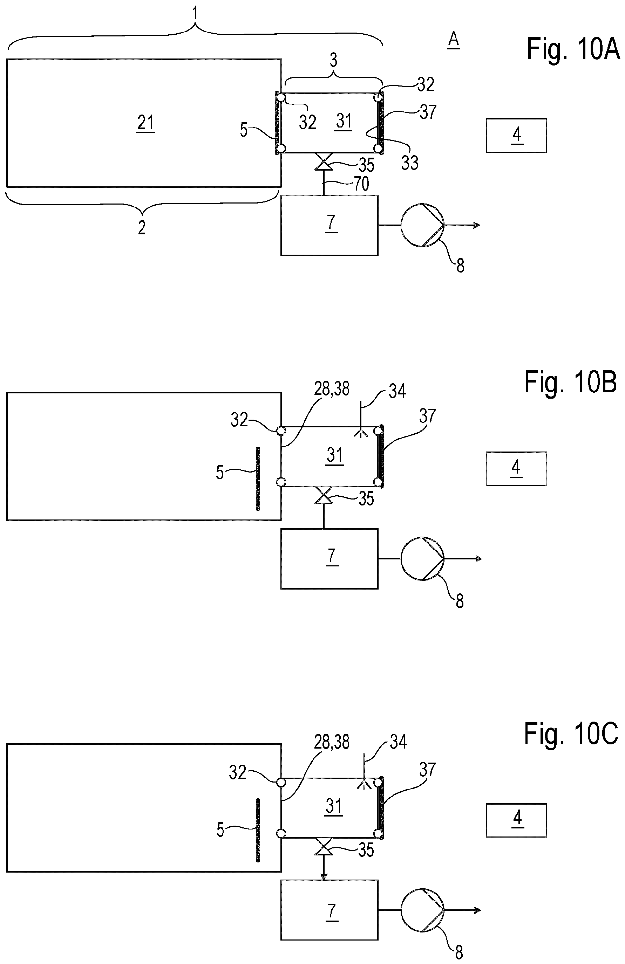

Figuren 10A bis 10H: der mittels einer Transfereinheit durchgeführte Prozessablauf zur Verarbeitung eines Objekts, mit Bewegungen des Objekts, Stellung von Eingangstor an der Schleuse und Transfertür am Übergang von der Schleuse zum Containment, mit Dekontaminations- und Spülvorgang, und zusätzlicher Dekontamination der Transfertür, in Schrittfolge mit Prinzipdarstellungen;
- Figur 10A -: Start: mit Schritt 1
- Figur 10B -: Schritt 2
- Figur 10C -: Schritt 3

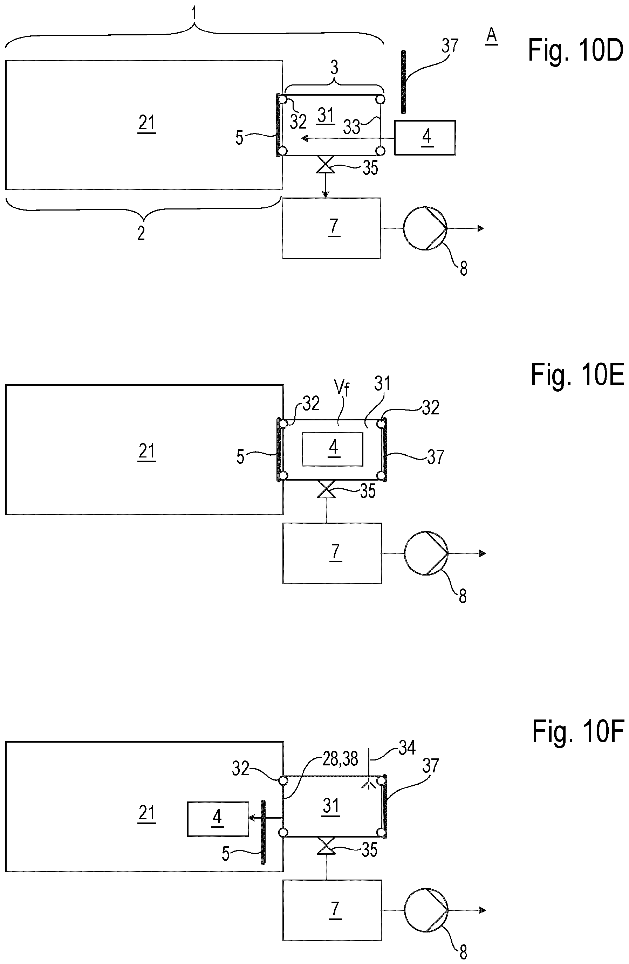

- Figur 10D -: Schritt 4
- Figur 10E -: Schritt 5
- Figur 10F -: Schritt 6; und

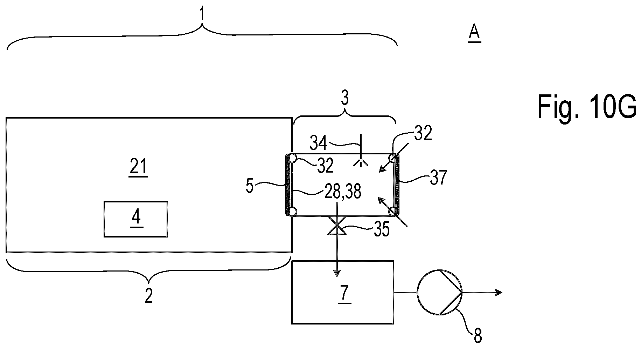

- Figur 10G -: Ende: mit Schritt 7

### Ausführungsbeispiel

Mit Bezug auf die beiliegenden Zeichnungen erfolgt nachstehend die detaillierte Beschreibung des erfindungsgemässen Verfahrens zum Überführen von Objekten unter aseptischen Bedingungen aus einer Schleuse in die von einem Gehäuse umgebene Prozesskammer eines Containments, das mit der Schleuse eine druckdicht miteinander verbundene Transfereinheit bildet.

Für die gesamte weitere Beschreibung gilt folgende Festlegung. Sind in einer Figur zum Zweck zeichnerischer Eindeutigkeit Bezugsziffern enthalten und dabei zeichnerisch eindeutig erkennbar ist, dass es sich um "wiederkehrende" Bauteile handelt, aber im unmittelbar zugehörigen Beschreibungstext nicht erläutert, so sei im Interesse der Verkürzung, auf deren Erklärung in vorangehenden Figurenbeschreibungen hingewiesen.

### Figur 1A

Die einzelne Transfereinheit **1** besteht aus dem Containment **2** und der daran angebauten Schleuse **3.** Zum Einbringen in die Schleuse **3** ist das zumindest eine Objekt **4** vorgesehen, das zunächst vor der verschlossenen Eingangstür der Schleuse **3** positioniert ist. Im Produktionsmodus steht eine Vielzahl von zu weiterverarbeitenden Objekten **4** bereit. Das einzelne Objekt **4** ist hier von wannenförmiger Gestalt, welches das Objektvolumen **V_{O}** besitzt. Von oben wird dem Objekt **4** eine gleichgerichtete Verdrängungsströmung **LF** zugeführt. Die Schleuse **3** ist vom Gehäuse **30** umgeben, wobei die umschlossene Schleusenkammer **31** das Schleusenvolumen **V_{S}** hat. Oben und unten am Gehäuse **30** ist jeweils ein Auslass **35** in Form eines einstellbaren Ventils montiert. Ferner ist oben am Gehäuse **30** ein Einlass **34** installiert, um darüber Dekontaminationsmittel in der entsprechenden Prozessphase einzubringen. Zur Schleuse **3** gehören ausserdem das im Gehäuse **30** eingebaute Eingangstor **37,** welches frei dem Aufstellraum **A** zugewandt ist, in dem sich der gesamte Aufbau befindet.

Am Übergang von der Schleuse **3** zum Containment **2,** welches vom Gehäuse **20** umgeben ist und darin die Prozesskammer **21** einschliesst, befindet sich auf Seiten der Schleuse **3** der Ausgang **38** und mit diesem im Prinzip deckungsgleich der in das Containment **2** mündende Zugang **28.** Im Ausgang **38** und dem Zugang **28** sitzt die Transfertür **5.** Oberhalb des Containments **2** sind hier zwei Zuluftfilter **23** angebaut, über die in die Prozesskammer **21** gleichgerichtete Verdrängungsströmung **LF** eingebracht wird. Unten am Gehäuse **20** des Containments **2** ist ein Auslass **25** in Form eines einstellbaren Ventils installiert.

### Figuren 1B und 1C

Ein einzelnes Objekt **4** umfasst:
- Ein Behältnis **40** mit einem aseptischen Innenraum **41** und einer obigen Öffnung **42,** die von einem Verschluss **46** verschlossen ist.
- Ein im Innenraum **41** gelagertes Nest **43,** in dessen muldenförmigen Aufnahmekonturen die Materialien **44** stecken, welche nach Öffnen des Verschlusses **46** in der Prozesskammer **21** des Containments **2** zu behandeln sind.
- Optional eine zwischen dem Verschluss **46** und dem Nest **43,** über den Materialien **44** eingefügte Einlage **45.**
- Im Anlieferungszustand ist das gesamte Objekt **4** zumeist von einer geschlossenen, beutelförmigen Umhüllung umgeben, die das Innenvolumen der Umhüllung steril hält.

Das Behältnis **40** ist z.B. ein wannenförmiges Tub. Die Materialien **44** sind insbesondere Phiolen, Vials oder Spritzen. Vom Verschluss **46** und der optionalen Umhüllung bestehen vorzugsweise zumindest Flächenanteile aus einem semipermeablen Vliesgewebe, wie Tyvek^{®}. Der Verschluss **46** ist typischerweise auf dem den Durchlass **42** des Behältnisses **40** umlaufenden Behältnisrandes **49** versiegelt und bewahrt so den Innenraum **41** des Behältnisses **40** in sterilem Zustand.

### Figuren 2A bis 2C

Diese Figurenfolge illustriert eine Transfereinheit **1** bestehend aus dem Containment **2** und einer daran andockbaren Schleuse **3** in drei verschiedenen Gestaltungen, je nach Anwendungsbedarf und Form der Behältnisse **40.** Das Containment **2** weist jeweils die Prozesskammer **21,** den Zugang **28** und die darin installierte Flanschanordnung **29** auf. An der jeweiligen Schleuse **3** befinden sich dem Zugang **28** und der Flanschanordnung **29** zugewandt, der Ausgang **38** und die Flanschanordnung **39.** Am anderen Ende haben Schleusen **3** das jeweilige Eingangstor **37.**

### Figuren 3A bis 3C

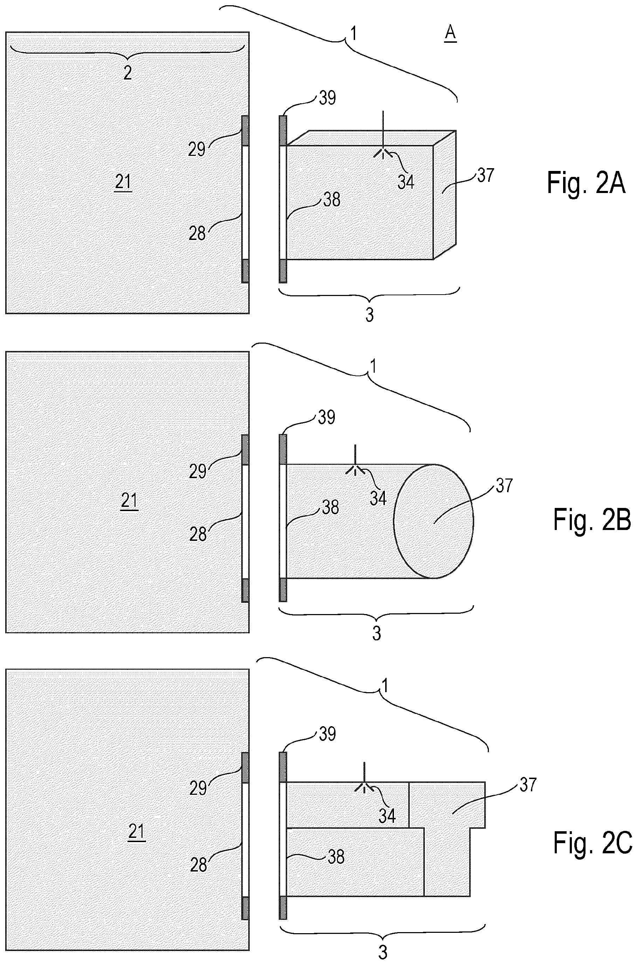

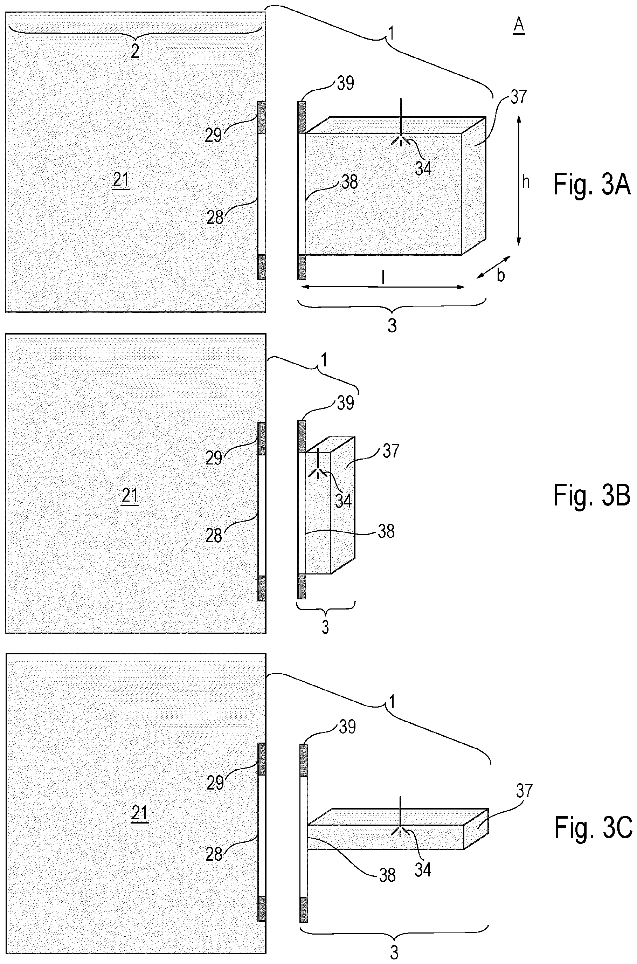

Bei den drei verschiedenen Gestaltungen der Schleuse **3** ist die Schleuse **3** gemäss Figur 2A mit Länge **I,** Breite **b** und Höhe **h** bemasst. Die Figuren 3B und 3C zeigen jeweils eine Transfereinheit **1,** bestehend aus dem Containment **2** und einer daran andockbaren Schleuse **3** in vierter und fünfter Gestalt. Bei den vorhandenen Bauteilen wird auf die Vorgängerfiguren 2A-2C Bezug genommen.

### Figur 4

Mit dem gezeigten Fliessschema soll der mittels der Transfereinheit **1** durchgeführte Prozessablauf zur Verarbeitung eines Objekts **4** dargestellt und beschrieben werden.

S_{O}: Steht für die Startphase, d.h. die zu verarbeitenden Objekte **4** sind definiert.

S₁: Entsprechend der Form der zu verarbeitenden Objekte **4** und der Chargengrösse - jeweils nur ein Objekt 4 oder mehrere davon - wird die Gestalt der an das Containment **2** andockbaren Schleuse **3** ausgewählt bzw. bei Einstellbarkeit von Schleusenkammer **31** und Schleusenvolumen **V_{S},** passend eingestellt.

S₂: Das bzw. die in einer Charge zu verarbeitenden Objekte **4** werden durch das geöffnete Eingangstor **37** in die Schleuse **3** geladen, evtl. mit der beutelförmigen Umhüllung um das Objekt **4.**

S₃: Bei geschlossenem Eingangstor **37** erfolgt die Dekontaminationsphase Eingangstor **37** mit der geeigneten Methode durch Versprühen, Vernebeln oder Verdampfen eines Dekontaminationsmittels, z.B. H₂O₂, oder durch Bestrahlung.

S₄: Der Zugang **28** in das Containment **2** wird geöffnet.

S₅: Das so vorbehandelte und zu transferierende Objekt **4** wird in das Containment **2** überführt. Eventuell kann auf die Spülphase verzichtet werden.

S₆: Eventuell am Objekt **4** vorhandene Reste an Dekontaminationsmittel werden in der Prozesskammer **21** des Containments **2** abgespült.

S₇: Die Weiterverarbeitung der der Objekte **4** in der Prozesskammer **21** des Containments **2** kann erfolgen, in der Regel, Öffnen des Verschlusses **46** und Entnahme der Materialien **44** zu deren nächsten Behandlungsschritten.

### Figuren 5A bis 5G

Anhand dieser Figurenfolge wird der mittels einer Transfereinheit **1** durchgeführte Prozessablauf zur Verarbeitung eines Objekts **4** mit Prinzipdarstellungen in der Schrittfolge gezeigt.

### Figur 5A - Start, Schritt 1

Es stehen das Containment **2,** die noch nicht daran angedockte Schleuse **3** sowie ein noch freistehendes Objekt **4** bereit, alles im Aufstellraum **A.** Geschlossen sind die Transfertür **5** am Containment **2** sowie das Eingangstor **37** und der Ausgang **38** der Schleuse **3.** Die Form und Grösse der Schleuse **3** wurde gemäss anstehender Aufgabe bestimmt.

### Figur 5B - Schritt 2

Containment **2** und Schleuse **3** sind aneinander gedockt, die dazwischenliegende Transfertür **5** ist geschlossen. Das Eingangstor **37** ist geöffnet und das Objekt **4** wird in die Schleusenkammer **31** eingefahren.

### Figur 5C - Schritt 3

Das Eingangstor **37** ist geschlossen und die Dekontaminationsphase wird durchgeführt. Durch Präsenz des Objekts **4** in der Schleusenkammer **31** verbleibt vom ursprünglichen Schleusenvolumen **V_{S},** jetzt nur mehr das freie Schleusenvolumen **V_{f}**.

### Figur 5D - Schritt 4

Die Transfertür **5** zwischen Containment **2** und Schleuse **3** wird geöffnet.

### Figur 5E - Schritt 5

Das Objekt **4** wird durch die Transfertür **5** aus der Schleusenklammer **31** in die Prozesskammer **21** des Containments **2** transferiert.

### Figur 5F - Schritt 6

Die Transfertür **5** wird geschlossen. Über den Einlass **34** an der Schleuse **3** erfolgt die Entgasung der Schleusenklammer **31.**

### Figur 5G - Ende, Schritt 7

In der Prozesskammer **21** des Containments **2** geschieht die Weiterverarbeitung des Objektes **4** bzw. der Charge von Objekten **4** eines Prozessdurchgangs.

### Figuren 6A bis 6F

Anhand dieser Figurenfolge wird der mittels einer Transfereinheit **1** durchgeführte Prozessablauf zur Verarbeitung eines Objekts **4** mit Bewegungen des Objekts **4** und Stellung von Eingangstor **37** an der Schleuse **3** und Transfertür **5** am Übergang von der Schleuse **3** zum Containment **2,** in der Schrittfolge beschrieben.

### Figur 6A - Start, Schritt 1

Das Eingangstor **37** an der Schleuse **3** und die Transfertür **5** sind geöffnet. Das Objekt 4 steht bereit, alles im Aufstellraum **A.**

### Figur 6B - Schritt 2

Das Eingangstor **37** an der Schleuse **3** wird geschlossen. Die Transfertür **5** bleibt geöffnet. Um auch die Dichtungen an der Transfertür **5** zu dekontaminieren, erfolgt eine kombinierte Dekontaminationsphase, in welcher von der Prozesskammer **21** und der Schleusenkammer **31** das gesamte Innere der dekontaminiert wird. Ebenso bei der anschliessenden Spülphase.

### Figur 6C - Schritt 3

In unveränderter Stellung von Eingangstor **37** und Transfertür **5** erfolgt eine Spülphase.

### Figur 6D - Schritt 4

Die Transfertür **5** wird geschlossen und anschliessend das Eingangstor **37** geöffnet, um das Objekt **4** in die Schleusenkammer **31** zu laden.

### Figur 6E - Schritt 5

Das Objekt **4** steht in der Schleusenkammer **31,** nun wird das Eingangstor **37** geschlossen und eine Dekontaminationsphase für die Schleusenkammer **31** und das darinstehende Objekt **4** durchgeführt, z.B. durch Einbringen des Dekontaminationsmittels über den Einlass **34.** Somit verbleibt durch die Präsenz des Objekts **4** in der Schleusenkammer **31** vom ursprünglichen Schleusenvolumen **V_{S},** jetzt nur mehr das freie Schleusenvolumen **V_{f}**.

### Figur 6F - Ende, Schritt 6

Die Transfertür **5** wird geöffnet, um das äusserlich dekontaminierte Objekt **4** von der Schleusenkammer **31** in die Prozesskammer **21** des Containments **2** zu transferieren und dort weiter zu bearbeiten.

### Figuren 7A und 7B

Dieses Figurenpaar zeigt den Zusammenbau von Schleuse **3** und Containment **2** in den Prinzipdarstellungen: Annäherung der Schleuse **3** an das Containment **2** (Figur 7A) und die Schleuse **3** ist lösbar an das Containment **2** angedockt (Figur 7B). Hierbei kommen der Zugang **29** am Containment **2** und Ausgang **38** der Schleuse **3** und in Übereinstimmung und die beiderseitigen Flanschanordnungen **29,39** werden miteinander verbunden.

### Figuren 8A und 8B

Dieses Figurenpaar zeigt den Zusammenbau von Schleuse **3** und Containment **2,** ausgestattet mit einem Medienblock **6,** in Prinzipdarstellungen: Annäherung der Schleuse **3** an das Containment **2,** Medienblock **6** und Anschluss **36** an der Schleusenkammer **31** noch getrennt (Figur 8A) und die Schleuse **3** an das Containment **2** lösbar angedockt, Medienblock **6** und Anschluss **36** miteinander verbunden (Figur 8B).

Beispielsweise oberhalb der Prozesskammer **21** ist eine Prozesstechnik **22** vorgesehen. An diese Prozesskammer **21** führen heran: eine Abluftleitung **60,** eine Pressluftleitung **61,** eine Druckluftleitung **62,** eine H₂O₂-Leitung **63,** ein Temperatur-Sensor **64,** ein Feuchte-Sensor **65,** Druck-Sensor **66,** ein H₂O₂-Sensor **67** mit Prozentangabe und eine Überdruckleitung **68.** Alle sind an einen Medienblock **6** angeschlossen. Im Verlauf der Abluftleitung **60** sind der Filter **600** und das Ventil **601** eingebaut. Im Verlauf der Pressluftleitung **61** sind der Filter **610** und das Ventil **611** eingebaut. Im Verlauf der Überdruckleitung **68** sitzt das Ventil **681.** Die Enden von Druckluftleitung **62** und H₂O₂-Leitung **63** führen zu einer Zweistoffdüse **69.** Im betriebsbereiten Zustand sind Containment **2** und Schleuse **3** mit ihren Flanschanordnungen **29,39** miteinander lösbar einander angedockt und der Medienblock **6** ist mit dem Anschluss **36** verbunden.

### Figuren 9A bis 9G

Anhand dieser Figurenfolge wird der mittels einer Transfereinheit **1** durchgeführte Prozessablauf zur Verarbeitung eines Objekts **4** mit Bewegungen des Objekts **4,** Stellung von Eingangstor **37** an der Schleuse **3** und Transfertür **5** am Übergang von der Schleuse **3** zum Containment **2,** nun mit Dekontaminations- und Spülvorgang, in der Schrittfolge beschrieben.

### Figur 9A - Start, Schritt 1

Äquivalent gemäss Beschreibung zu Figur 6A.

### Figur 9B - Schritt 2

Äquivalent gemäss Beschreibung zu Figur 6B.

### Figur 9C - Schritt 3

Äquivalent gemäss Beschreibung zu Figur 6C.

### Figur 9D - Schritt 4

Äquivalent gemäss Beschreibung zu Figur 6D.

### Figur 9E - Schritt 5

Äquivalent gemäss Beschreibung zu Figur 6E.

### Figur 9F - Schritt 6

Die Transfertür **5** wird geöffnet, um das äusserlich dekontaminierte Objekt **4** von der Schleusenkammer **31** in die Prozesskammer **21** des Containments **2** zu transferieren und dort weiter zu bearbeiten. Während dieser Phase kann ein Spülvorgang stattfinden, z.B. durch Hin- und Herbewegen des Objekts **4,** ähnlich eines alternierenden Pumpenkolbens oder durch extern zugeführte und nach aussen abgeleitete Spülluft.

### Figur 9G - Ende, Schritt 8

Bei dieser Alternative liegt das äusserlich dekontaminierte Objekt **4** in der Prozesskammer **21** und die Transfertür **5** ist geschlossen. Der Zugang **33** in die Schleusenkammer **31** ist durch Öffnen der Dichtung **32** am Eingangstor **37** für Frischluftzufuhr **L_{f}** strömungsdurchlässig. Mittels Anschlusses eines Vakuums **L_{V}** an die Schleusenkammer **31** wird eine Spülphase initiiert, der Luftzustrom erfolgt durch den Strömungsdurchlas am Eingangstor **37.** Ein nächster Verfahrensdurchlauf gemäss Figur 9D kann beginnen.

### Figuren 10A bis 10G

Basierend auf dieser Figurenfolge wird der mittels einer Transfereinheit **1** durchgeführte Prozessablauf zur Verarbeitung eines Objekts **4** mit Bewegungen des Objekts **4,** Stellung von Eingangstor **37** an der Schleuse **3** und Transfertür **5** am Übergang von der Schleuse **3** zum Containment **2,** mit Dekontaminations- und Spülvorgang, und zusätzlicher Dekontamination der Transfertür **5,** in der Schrittfolge beschrieben.

### Figur 10A - Start, Schritt 1

Es stehen das Containment **2** und die daran angedockte Schleuse **3** sowie ein noch freistehendes Objekt **4** bereit, alles im Aufstellraum **A.** Die Form und Grösse der Schleuse **3** wurden wiederum gemäss anstehender Aufgabe bestimmt. In Modifikation zum bisherigen Aufbau der Transfereinheit **1,** liegt zwischen der Prozesskammer **21** des Containments **2** und der Schleusenkammer **31** die verstellbare Transfertür **5,** deren Abdichtung mittels einer vorzugsweise pneumatisch betätigbaren Dichtung **32** erfolgt, so dass bei geöffneter Transfertür **5** der Zugang **28** und der Ausgang **38** offen sind. Der Zugang **33** in die Schleusenkammer **31** ist durch das Eingangstor **37** verschliessbar, wobei der Verschluss vorzugsweise mittels einer pneumatisch betätigbaren Dichtung **32** erfolgen kann. Aus der Schleusenkammer **31** erstreckt sich eine pneumatische Leitung **70** zu einem Gasbehälter **7,** wobei in der Leitung **70** ein Auslassventil **35** installiert ist und an den Gasbehälter **7** eine Vakuumpumpe **8** angeschlossen ist.

### Figur 10B - Schritt 2

Das Eingangstor **37** an der Schleuse **3** bleibt geschlossen und die z.B. pneumatische Dichtung **32** ist aktiviert. Die Transfertür **5** wird geöffnet und die z.B. pneumatische Dichtung **32** ist deaktiviert. Um auch die Dichtungen **32** an der Transfertür **5** zu dekontaminieren, erfolgt eine kombinierte Dekontaminationsphase, die gleichzeitig in die Prozesskammer **21** hineinwirkt, z.B. durch Einbringen des Dekontaminationsmittels H₂O₂ über den Einlass **34** in die Schleusenkammer **31.** Vorteilhaft können dazu am Containment **2** Dekontaminationsvorrichtungen, z.B. mehrere Zweistoffdüsen **69** (äquivalent zu Figur 8A) installiert sein, um H₂O₂ einzutragen. Durch den geschlossenen Auslass **35,** als Ventil, kann über die Leitung **70** kein Gasvolumen in den Gasbehälter **7** von der Vakuumpumpe **8** abgesaugt werden, welches als Dekontaminationsmittel über den Einlass **34** in die Schleusenkammer **31** strömte. In der Prozesskammer **21** und der Schleusenkammer **31** entsteht ein Überdruck.

### Figur 10C - Schritt 3

Mit Öffnen des Auslasses **35** wird über die Leitung **70** Gasvolumen aus der Prozesskammer **21** und der Schleusenkammer **31** in den Gasbehälter **7** von der Vakuumpumpe **8** abgesaugt, was als Spülphase wirkt. Die Spülluft wird vorzugsweise über den Einlass **34** zugeführt. Vorteilhaft trägt zur Spülphase auch die am Containment **2** vorhandene Lüftungstechnik bei.

### Figur 10D - Schritt 4

Zuerst wird die Transfertür **5** geschlossen, dann das Eingangstor **37** geöffnet, um das Objekt **4** in die Schleusenkammer **31** zu laden. Der Auslass **35** bleibt geöffnet und das Absaugen über den Gasbehälter 7 mittels der Vakuumpumpe **8** wird fortgesetzt.

### Figur 10E - Schritt 5

Die Transfertür **5** bleibt geschlossen, der Auslass **35** und das Eingangstor **37** werden geschlossen. Nun erfolgt eine Dekontaminationsphase der Schleusenkammer **31** mit dem darin befindlichen Objekt **4,** z.B. durch Einbringen des Dekontaminationsmittels H₂O₂ über den Einlass **34.** Somit verbleibt durch die Präsenz des Objekts **4** in der Schleusenkammer **31** vom ursprünglichen Schleusenvolumen **V_{S},** jetzt nur mehr das freie Schleusenvolumen **V_{f}**.

### Figur 10F - Schritt 6

Die Transfertür **5** wird geöffnet, der Auslass **35** und das Eingangstor **37** bleiben geschlossen. Nun erfolgt der Transfer des äusserlich dekontaminierten Objekts **4** von der Schleusenkammer **31** in die Prozesskammer **21** des Containments **2.**

### Figur 10G - Schritt 7

Diese Figur illustriert das beschleunigte Spülen der Schleusenkammer **31** mittels Vakuums. Dazu wird der Auslass **35** geöffnet und die pneumatische Dichtung **32** am Eingangstor **37** deaktiviert, so dass Luft aus dem Aufstellraum **A** in die Schleusenkammer **31** nachfliessen kann. Das während der Spülphase entstehende Gasvolumen wird den offenen Auslass **35** durch den Gasbehälter **7** von der Vakuumpumpe **8** abgesaugt. Ein nächster Verfahrensdurchlauf gemäss Figur 10D kann beginnen.

## Patentansprüche

1. Verfahren zum Überführen von Objekten **(4)** unter aseptischen Bedingungen aus einer Schleuse **(3)** in die von einem Gehäuse **(20)** umgebene Prozesskammer **(21)** eines Containments **(2),** das mit der Schleuse **(3)** eine druckdicht miteinander verbundene Transfereinheit **(1)** bildet, wobei:
a) ein Gehäuse **(30)** der Schleuse **(3)** eine Schleusenkammer **(31)** mit dem Schleusenvolumen **(V_{S})** umgibt;
b) die Schleusenkammer **(31)** besitzt:
ba) ein druckdicht verschliessbares Eingangstor **(37),** welches im offenen Zustand einen Zugang **(33)** vom äusseren Aufstellraum **(A)** zum Einbringen von Objekten **(4)** in die Schleusenkammer **(31)** erlaubt;
bb) einen Ausgang **(38),** der zum Überführen der Objekte **(4)** aus der Schleusenkammer **(31)** in die Prozesskammer **(21)** bestimmt ist;
bc) einen Einlass **(34)** zum Einbringen von Dekontaminationsmittel;
c) das Gehäuse **(20)** einen Zugang **(28)** in die Prozesskammer **(21)** aufweist;
d) der Ausgang **(38)** aus der Schleusenkammer **(31)** in den Zugang **(28)** in die Prozesskammer **(21)** mündet;
e) zwischen dem Ausgang **(38)** aus der Schleusenkammer **(31)** und dem Zugang **(28)** in die Prozesskammer **(21)** eine verschliessbare Transfertür **(5)** vorgesehen ist;
f) ein einzelnes Objekt **(4)** umfasst:
fa) ein Behältnis **(40)** mit einem aseptischen Innenraum **(41)** und einer Öffnung **(42),** die mit einem Verschluss **(46)** verschlossen ist; und
fb) im Innenraum **(41)** gelagertes Material **(44),** welches nach Aufmachen des Verschlusses **(46)** in der Prozesskammer **(21)** zu behandeln ist, **gekennzeichnet durch** folgende Verfahrensschritte:
g) Bestimmung von Dimension und Form der Schleusenkammer **(31)** adäquat zum äusseren Objektvolumen **(V_{O})** eines einzelnen oder mehrerer Objekte **(4)** mit dem Ziel der Minimierung des **durch** das eine oder mehrerer im Schleusenvolumen **(V_{S})** liegenden Objekte **(4)** verbleibenden freien Schleusenvolumens (**V_{f}**);
h) Einbringen eines einzelnen oder mehrerer Objekte **(4)** in die ausgewählte Schleusenkammer **(31) durch** das offene Eingangstor **(37)** bei geschlossener Transfertür **(5);**
i) Einleiten von Dekontaminationsmittel über den Einlass **(34)** in die Schleusenkammer **(31)** bei geschlossenem Eingangstor **(37);**
j) optionales Spülen der Schleusenkammer **(31)** und Ableiten von Spülmittel aus der Schleusenkammer **(31)** über einen Auslass **(35)** nach erfolgreicher Dekontamination; und
k) Überführen des einzelnen oder mehrerer äusserlich dekontaminierter Objekte **(4) durch** die geöffnete Transfertür **(5)** aus der Schleusenkammer **(31)** in die Prozesskammer **(21).**

2. Verfahren nach Anspruch 1, **dadurch gekennzeichnet, dass** aufgrund des geringen verbleibenden freien Schleusenvolumens **(V_{f})** die Dekontaminationsphase zeitlich kurzgehalten werden kann, somit das Diffundieren von Dekontaminationsmittel in den Innenraum **(41)** des Objekts **(4)** minimiert wird und die Verarbeitung des Objekts **(4)** im Containment **(2)** rasch erfolgt.

3. Verfahren nach zumindest einem der Ansprüche 1 oder 2, **dadurch gekennzeichnet, dass** die Verbindung zwischen der Schleuse **(3)** und dem Containment **(2)** mittels beiderseits vorhandener Flanschanordnungen **(29,39)** erfolgt, welche vorzugsweise druckdicht ausgestaltet sind.

4. Verfahren nach zumindest einem der Ansprüche 1 bis 3, **dadurch gekennzeichnet, dass** das Dekontaminationsmittel in der Schleusenkammer **(31)** zerstäubt wird, z.B. mittels einer Zweistoffdüse **(69)** oder durch Ultraschall, und vorzugsweise flüssiges H₂O₂ ist.

5. Verfahren nach zumindest einem der Ansprüche 1 bis 4, **dadurch gekennzeichnet, dass** optionale Spülen der Schleusenkammer **(31)** durch alternierenden Fluss in wechselnder Strömungsrichtung aus den Gasvolumen zwischen der Prozesskammer **(21)** und der Schleusenkammer **(31)** erfolgt.

6. Verfahren nach zumindest einem der Ansprüche 1 bis 5, **dadurch gekennzeichnet, dass** die Transfertür **(5)** am Ausgang **(38)** aus der Schleusenkammer **(31)** oder am Zugang **(28)** in das Containment **(2)** vorgesehen ist.

7. Verfahren nach zumindest einem der Ansprüche 1 bis 6, **dadurch gekennzeichnet, dass** die an der Schleuse **(3)** und am Containment **(2)** vorhandenen Flanschanordnungen **(29,39)** als Schnittstelle beschaffen sind, wodurch das Andocken verschieden dimensionierter Schleusen **(3)** unterschiedlicher Formen an das Containment **(2)** ermöglicht wird.

8. Verfahren nach zumindest einem der Ansprüche 1 bis 7, **dadurch gekennzeichnet, dass** das Überführen des einzelnen oder mehrerer äusserlich dekontaminierter Objekte **(4)** aus der Schleusenkammer **(31)** in die Prozesskammer **(21)** nach teilweiser oder vollständiger Spülung der Schleusenkammer **(31)** erfolgt.

9. Verfahren nach zumindest einem der Ansprüche 1 bis 8, **dadurch gekennzeichnet, dass** vor dem Beladen der Schleuse **(3)** bei geschlossenem Eingangstor **(37)** und offener Transfertür **(5)** die Schleusenkammer **(31)** und die Prozesskammer **(21)** sowie die Dichtflächen an der Transfertür **(5)** dekontaminiert werden.

10. Verfahren nach zumindest einem der Ansprüche 1 bis 9, **dadurch gekennzeichnet, dass** die Schleuse **(3)** einen Anschluss **(36)** besitzt, um daran einen Medienblock **(6)** anzuschliessen, welcher mit einer Prozesstechnik **(22)** verbunden ist, die Bestandteil des Containments **(2)** oder der Schleuse **(3)** sein kann oder unabhängig davon positioniert ist.

11. Verfahren nach Anspruch 10, **dadurch gekennzeichnet, dass** der Medienblock **(6)** Leitungen für Druckluft **(62)** und/oder Dekontaminationsmittel **(63),** und/oder Sensoren für Temperatur **(64),** Feuchte **(65),** Druck **(66),** H₂O₂-Konzentration **(67)** und Prozess- und Abluft **(61,60)** aufweist.

12. Verfahren nach zumindest einem der Ansprüche 1 bis 11, **dadurch gekennzeichnet, dass** das optionale Spülen mittels Druckluft **(62)** und/oder Prozessluft **(61)** erfolgt, welche über die Umgebungstemperatur im Aufstellraum **(A)** hinaus aufgewärmt sein kann.

13. Verfahren nach zumindest einem der Anspruch 11 oder 12, **dadurch gekennzeichnet, dass** die Verbindung zwischen dem Anschluss **(36)** an der Schleuse **(3)** und dem Medienblock **(6)** mechanisch lösbar oder unlösbar oder pneumatisch gekoppelt beschaffen ist.

14. Verfahren nach zumindest einem der Ansprüche 1 bis 13, **dadurch gekennzeichnet, dass** nach Einbringen eines einzelnen oder mehrerer Objekte **(4)** in die Schleusenkammer **(31),** gemäss deren gewählter Dimensionierung, das vom Schleusenvolumen **(V_{S})** verbleibende freie Schleusenvolumen (**V_{f}**) zwischen 10% und 80%, vorzugsweise zwischen 20% und 60%, insbesondere zwischen 30% und 50% beträgt.

15. Verfahren nach zumindest einem der Ansprüche 1 bis 14, **dadurch gekennzeichnet, dass** die Anpassung der Dimensionierung des Schleusenvolumen **(V_{S})** an das einzubringende einzelne oder mehrerer Objekte **(4),** mit der Zielstellung der Minimierung des verbleibenden freien Schleusenvolumens **(V_{f})** auf zwischen 10% und 80%, vorzugsweise auf zwischen 20% und 60%, insbesondere auf zwischen 30% und 50% durch einengendes Verschieben von Wandungen an der Schleusenkammer **(31)** und/oder durch Einbringen eines Volumen verdrängenden Füllkörpers realisiert wird.

16. Verfahren nach zumindest einem der Ansprüche 1 bis 15, **dadurch gekennzeichnet, dass** das Objekt **(4)** die Gestalt eines Gebindes mit seinem äusseren Objektvolumen **(V_{O})** hat und umfasst:
a) ein Behältnis **(40)** mit einem aseptischen Innenraum **(41)** und einer Öffnung **(42),** die mit einem Verschluss **(46),** vorzugsweise in Gestalt einer Abdeckung, verschlossen ist; und
b) im Innenraum **(41)** gelagertes Material **(44)** in Form von Phiolen oder medizinischen Spritzen, welche nach Aufmachen des Verschlusses **(46)** in der Prozesskammer **(21)** zu behandeln sind, z.B. mit pharmazeutischen Substanzen zu befüllen sind.

17. Verfahren nach zumindest einem der Ansprüche 1 bis 15, **dadurch gekennzeichnet, dass** das Objekt **(4)** die Gestalt einer Flasche, z.B. einer Zellkulturflasche, oder eines Beutels - z.B. mit darin enthaltenen Agarplatten - oder einer Ampulle hat, welche ein äusseres Objektvolumen **(V_{O})** bilden und umfasst:
a) einen aseptischen Innenraum **(41)** und einer vorhandenen Öffnung **(42),** die mit einem Verschluss **(46)** verschlossen ist oder erst zu schaffen ist; und
b) im Innenraum **(41)** gelagertes Material **(44),** welches nach Aufmachen des Verschlusses **(46)** in der Prozesskammer **(21)** zu behandeln ist; oder
c) den zu befüllenden Innenraum **(41),** z.B. mit pharmazeutischen Substanzen.

18. Verfahren nach zumindest einem der Ansprüche 1 bis 17, **dadurch gekennzeichnet, dass** das optionale Spülen der Schleusenkammer **(31)** bei offener Transfertür **(5)** durch mindestens ein einmaliges und zumindest partielles Einfahren des Objekts **(4)** aus der Prozesskammer **(21)** des Containments **(2)** in die Schleusenkammer **(31)** erfolgt.

19. Verfahren nach zumindest einem der Ansprüche 1 bis 18, **dadurch gekennzeichnet, dass** zur Beschleunigung des Spülens der Schleusenkammer **(31)** eine an diese aktivierbare Vakuumpumpe **(8)** angeschlossen ist.

20. Verfahren nach Anspruch 19, **dadurch gekennzeichnet, dass** bei beschleunigtem Spülen der Schleusenkammer **(31):**
a) zuerst ein an die Schleusenkammer **(31)** angeschlossener Auslass **(35)** geöffnet wird, der zu einem Gasbehälter **(7)** führt; und anschliessend
b) die an den Gasbehälter **(7)** angeschlossene Vakuumpumpe **(8)** aktiviert wird; oder
c) zuerst die an den Gasbehälter **(7)** angeschlossene Vakuumpumpe **(8)** aktiviert wird; und anschliessend;
d) ein an die Schleusenkammer **(31)** angeschlossener Auslass **(35)** geöffnet wird, der zu einem Gasbehälter **(7)** führt.

21. Verfahren nach zumindest einem der Ansprüche 19 und 20, **dadurch gekennzeichnet, dass** bei beschleunigtem Spülen der Schleusenkammer **(31):**
a) zuerst der an die Schleusenkammer **(31)** angeschlossene Auslass **(35)** geöffnet wird, der zu einem Gasbehälter **(7)** führt; und anschliessend
b) die an den Gasbehälter **(7)** angeschlossene Vakuumpumpe **(8)** aktiviert wird; und
c) dann zum Druckausgleich zwischen der Prozesskammer **(21)** und der Schleusenkammer **(31)** die Dichtung **(32)** an der Transfertür **(5)** gelöst wird;
oder
d) zuerst die an den Gasbehälter **(7)** angeschlossene Vakuumpumpe **(8)** aktiviert wird; und anschliessend
e) der an die Schleusenkammer **(31)** angeschlossene Auslass **(35)** geöffnet wird, der zu einem Gasbehälter **(7)** führt; und
f) dann zum Druckausgleich zwischen der Prozesskammer **(21)** und der Schleusenkammer **(31)** die Dichtung **(32)** an der Transfertür **(5)** gelöst wird.

22. Verfahren nach Anspruch 21, **dadurch gekennzeichnet, dass** das Lösen der Dichtung **(32)** an der Transfertür **(5)** zwecks Druckausgleichs zwischen Prozesskammer **(21)** und Schleusenkammer **(31)** durch Deaktivierung einer pneumatischen Dichtung **(32)** erfolgt.
